# NOUVEAU FASCICULE DE BREVET EUROPEEN

(11) **EP 0 472 713 B2**
(45) Date de publication et mention de la décision concernant l'opposition: **02.05.2003**
(45) Mention de la délivrance du brevet: 20.08.1997
(21) Numéro de dépôt: 91906634.0
(22) Date de dépôt: 13.03.1991
(51) Int. Cl.: G01N 21/78, G01N 33/22

(54) **PROCEDES COLORIMETRIQUES DE DETERMINATION ET DE REGULATION DE LA TENEUR EN PERACIDE DANS UNE SOLUTION, EN PRESENCE DE PEROXYDE D'HYDROGENE**
KOLORIMETRISCHE VERFAHREN ZUR BESTIMMUNG UND REGELUNG DES PERSÄUREGEHALTS EINER LÖSUNG IN GEGENWART VON WASSERSTOFFPEROXID
COLORIMETRIC PROCESSES FOR DETERMINING AND ADJUSTING THE PERACID CONTENT OF A SOLUTION IN THE PRESENCE OF HYDROGEN PEROXIDE

(30) Priorité: 16.03.1990 FR 9003374
(43) Date de publication de la demande: 04.03.1992
(73) Titulaire: L'air Liquide, S.A. à Directoire et Conseil de Surveillance pour l'Etude et l'Exploitation des Procédés Georges Claude, 75321 Paris Cedex 07 (FR)
(72) Inventeur: MALLARD DE LA VARENDE, Jean, F-92100 Boulogne (FR); CRISINEL, Pascal, F-78000 Versailles (FR)
(74) Mandataire: Conan, Philippe Claude
(86) Numéro de dépôt international: FR9100203
(87) Numéro de publication internationale: WO91014172

(56) Documents cités:
- EP-A- 0 150 123
- EP-A- 0 239 468
- EP-A- 0 322 631
- US-A- 3 485 588
- ANALYST, volume 113, Septembre 1988 D.M. DAVIS et al. "Determination of Peracids in the Presence of a Large Excess of Hydrogen Peroxide Using a Rapid and Convenient Spectrophotometric Method"

## Description

L'invention concerne la détermination et la régulation de la teneur en peracide en présence de peroxyde d'hydrogène dans une solution oxydante, notamment une solution de nettoyage et/ou de désinfection utilisable notamment à l'échelle industrielle.

Pour le nettoyage et/ou la désinfection d'installations industrielles, notamment en laiterie, on utilise couramment des solutions oxydantes comprenant un peracide, notamment l'acide peracétique (APA) et du peroxyde d'hydrogène, éventuellement en présence d'autres composés tels que notamment l'acide acétique et/ou l'acide nitrique.

Ces solutions sont habituellement utilisées en circuit fermé, dans une canalisation principale faisant notamment partie de l'installation à nettoyer et/ou désinfecter.

Elles peuvent être utilisées dans un système avec récupération de la solution après utilisation ou dans un système sans récupération de la solution après utilisation mais dans lequel on vérifie, à intervalles plus ou moins réguliers, la composition de la solution et on l'ajuste si nécessaire.

Notamment dans le cas d'une utilisation pour la désinfection, il est important de s'assurer que la solution utilisée présente une composition telle qu'elle soit active contre les microorganismes à détruire. Dans ce contexte, il convient donc de contrôler au moins la concentration de la substance la plus active, en l'occurence le peracide, qui est le plus souvent l'acide peracétique.

Dans la pratique industrielle actuelle, on procède à un dosage manuel du peracide au moment de l'utilisation. Ce dosage permet de déterminer la concentration exacte en peracide (et également si nécessaire en peroxyde d'hydrogène) mais il est laborieux et nécessite le recours à de la main-d'oeuvre spécialisée, de plus il n'est pas automatisable et ne permet pas une régulation en continu de la teneur en peracide.

On a donc cherché à automatiser ce dosage en ayant recours à un moyen simple et peu onéreux qui permette également une régulation "en temps réel" de la concentration en peracide, notamment en acide peracétique.

Pour ce faire, on a déjà proposé un certain nombre de méthodes et dispositifs qui sont décrits et commentés brièvement ci-après.

La demande de brevet FR 2 595 837 (HENKEL FRANCE S.A.) propose une solution qui est essentiellement basée sur une analyse potentiométrique dans une cellule de mesure comportant une électrode de mesure du potentiel rédox de la solution oxydante et une électrode de mesure du pH de cette solution. Pour obtenir une lecture significative, c'est-à-dire des points de mesure toujours sur la même courbe concentration/potentiel d'oxydo-réduction dans une zone dans laquelle la variation est rapide, éviter la polarisation de l'électrode de mesure et travailler à pH constant, la solution proposée utilise un circuit dérivé permettant une dilution du flux au moyen d'une "solution d'adaptation". Dans le cas d'une solution contenant du peroxyde d'hydrogène et un peracide, l'analyse rend essentiellement compte de la concentration en peroxyde d'hydrogène et non de celle du composé véritablement actif, à savoir le peracide, notamment l'acide peracétique.

Par ailleurs, d'autres méthodes utilisées, à savoir la pH-métrie et la conductimétrie donnent une indication quant à l'acidité du milieu mais ne donnent pas directement d'informations en ce qui concerne l'évolution de l'oxydant essentiel, à savoir le peracide.

On a en outre proposé, pour analyser le pouvoir désinfectant des solutions du type en question, d'avoir recours à la colorimétrie qui permet de différencier le peroxyde d'hydrogène d'un peracide.

Ainsi, la demande de brevet EP 0 150 123 (INTE-ROX CHEMICALS LIMITED) décrit un procédé d'analyse et de régulation de la teneur en un composé peroxydé, notamment l'acide peracétique, en présence de peroxyde d'hydrogène, en ayant recours à des méthodes colorimétriques. Ce procédé repose sur le fait qu'il est connu que le peroxyde d'hydrogène réagit avec les réactifs colorés à la température ambiante, alors que les composés peroxydés, en particulier l'acide peracétique, ne réagissent pas d'une manière significative avec ces réactifs colorés mais nécessitent un chauffage jusqu'à une température de par exemple 90°C pour développer complètement une couleur. Le procédé décrit utilise donc deux circuits de mesure dont l'un est chauffé. La concentration en composé peroxydé est obtenue à partir de la différence d'intensité des couleurs développées dans ces deux circuits, l'une étant due au peroxyde d'hydrogène seul et l'autre à l'ensemble peroxyde d'hydrogène-composé peroxydé. Cette façon de procéder qui implique le chauffage de l'un des circuits présente le désavantage d'être compliquée. De plus et surtout, elle donne souvent une mesure imprécise de la concentration en composé peroxydé dans les solutions industrielles habituellement utilisées car la différence lue a une valeur relative faible, étant donné que ces solutions comportent en général une concentration en composé peroxydé, tel que notamment l'acide peracétique, très inférieure à la concentration en peroxyde d'hydrogène.

Il convient en outre de noter que bien que la demande de brevet EP 0 150 123 fasse allusion page 3, lignes 28 à 32, à l'utilisation possible de réactifs colorimétriques variés dont notamment les iodures, elle vise plus particulièrement l'utilisation de l'oxalate de potassium et de titane et surtout du molybdate de sodium (voir notamment page 3, lignes 27 et 37 et revendication 4). Au surplus, il convient de noter que les iodures ne pourraient pas être utilisés dans ce procédé car, comme il sera vu plus loin, ils réagissent très bien avec les peracides à la température ambiante, ce qui en revanche n'est pas le cas pour le peroxyde d'hydrogène.

Par ailleurs, la demande de brevet EP 0 322 631 (MERCK PATENT GmbH) décrit un procédé et un réactif pour la détermination des peracides, en particulier de l'acide peracétique, en présence de peroxyde d'hydrogène. Le réactif utilisé est constitué d'un chromogène, d'un iodure et éventuellement d'un tampon. Dans ce procédé, on ne mesure pas la coloration de l'iode formé au cours de la réaction d'oxydo-réduction mais la coloration due au chromogène. Comme il est indiqué page 3, lignes 6 à 8, la concentration en iodure peut être très faible, par exemple de l'ordre de un dizième de la concentration en chromogène car au cours de la réaction rédox l'iodure est d'abord consommé mais est ensuite de nouveau formé; il joue donc ici un rôle uniquement catalytique. La concentration en peracide, notamment en acide peracétique, est déterminée à l'aide d'une courbe d'étalonnage. Ce document concerne un procédé de dosage et un réactif approprié mais ne décrit ni ne suggère leur utilisation dans la régulation en continu de la teneur en peracide d'une solution.

Il est par ailleurs connu qu'à température ambiante, l'acide peracétique réagit presque instantanément avec les iodures pour donner de l'iode, alors que dans ces conditions le peroxyde d'hydrogène ne réagit que très lentement, voire même pas du tout. Cette particularité de réaction des peracides avec les iodures a donné lieu à différentes études rapportées dans la littérature. Récemment, D. Martin Davies et Michael E. Deary ont proposé, dans Analyst (London), septembre 1988, vol 113, p. 1477-1479, une méthode spectrophotométrique de détermination des péracides en présence d'un grand excès de peroxyde d'hydrogène qui utilise cette particularité. Cette méthode présentée comme simple et rapide est en fait complexe puisqu'elle implique, pour déterminer chaque concentration du peracide, l'établissement d'une courbe d'absorption en fonction du temps, après ajout de l'iodure à l'échantillon prélevé, et la concentration en question est obtenue par extrapolation linéaire au temps t=0 d'ajout de l'iodure.

On a maintenant trouvé selon l'invention qu'il était possible de réguler de façon simple la teneur en peracide, notamment en acide peracétique, en présence de peroxyde d'hydrogène, selon des procédés basés sur la colorimétrie et qui ne nécessitent pas l'utilisation de deux circuits de mesure dont l'un est chauffé, avec les inconvénients mentionnés ci-dessus à propos du système décrit dans la demande de brevet EP 0 150 123, en utilisant la cinétique différente de réaction du peroxyde d'hydrogène et des peracides avec les iodures exposés plus haut, sans avoir à établir une courbe particulière pour chaque détermination, comme dans le cas du procédé décrit dans Analyst et commenté ci-dessus.

Selon l'invention on a en effet établi qu'il était possible d'obtenir, par analyse colorimétrique à la température ambiante, de mélanges peroxyde d'hydrogène-peracide dans lesquels le rapport molaire de la concentration en peroxyde d'hydrogène à la concentration en peracide ne dépasse pas environ 100, une réponse linéaire de la coloration lue en fonction de la concentration en peracide, après ajout d'un excès d'iodure, pour un temps déterminé de réaction. La lecture de la concentration peut alors se faire directement sur une courbe d'étalonnage réalisée à partir de deux points, ou sur un appareil approprié, étalonné également à partir de deux « points ».

L'invention a pour objet un procédé colorimétrique de régulation de la teneur en peracide en présence de peroxyde d'hydrogène dans une solution au moins en partie aqueuse, dans laquelle le rapport molaire de la concentration en peroxyde d'hydrogène à la concentration en peracide ne dépasse pas environ 100, caractérisé en ce que :
- le peracide est l'acide peracétique,
- la solution à réguler est aspirée par un premier moyen d'aspiration, notamment une pompe péristaltique, dans les circuits d'aspiration d'un deuxième et d'un troisième moyen d'aspiration, notamment des pompes péristaltiques, fonctionnant au même débit et pouvant aspirer simultanément un liquide de dilution et/ou de réglage du pH, le deuxième moyen d'aspiration alimentant la cuve de référence d'un photomètre bi-faisceaux et le troisième moyen d'aspiration alimentant la cuve de mesure dudit photomètre,
- les débits des trois moyens d'aspiration sont réglés de telle sorte que la concentration en peracide dans le flux de mesure aspiré par lesdits deuxième et troisième moyens d'aspiration soit inférieure à environ 100 ppm (parties par million) en poids, et que le pH du flux de mesure soit inférieur à 6,5,
- le flux de mesure sortant du troisième moyen d'aspiration est additionné d'un excès d'iodure à l'aide d'un quatrième moyen d'aspiration, notamment une pompe péristaltique,
- les cellules du photomètre sont reliées à une unité de mesure qui compare l'intensité de la couleur d'iode formé dans la cuve de mesure à la référence dans la cuve de référence et transfere la différence à un régulateur, lequel régulateur actionne, si nécessaire, une pompe d'injection d'une solution de peracide dans le milieu à réguler, et
- la mesure est effectuée dans une gamme de longueur d'onde comprise entre 430nm et 500nm

L'acide peracétique est très couramment utilisé pour la désinfection des installations industrielles, notamment en laiterie.

La possibilité d'effectuer la détermination au voisinage de la température ambiante, c'est-à-dire à une température de 5 à 40°C environ, présente un avantage certain par rapport à l'art antérieur tel que représenté notamment par la demande de brevet EP 0 150 123. Ceci étant, la température ne doit si possible pas dépasser 50°C, car alors la mesure serait moins précise, le peroxyde d'hydrogène présent dans le milieu commençant alors à réagir de façon non négligeable avec l'iodure pour former de l'iode.

Il est bien connu que l'oxydation d'un iodure en iode ne peut avoir lieu qu'en milieu acide. On a à ce propos constaté que l'on n'obtient des résultats satisfaisants qui si le pH, au moment de la détermination, est inférieur ou égal à 6,5.

Des résultats satisfaisants sont également obtenus dans des solutions très acides (pH = 1) mais cela ne présente en général pas d'intérêt car les solutions habituellement dosées ont des pH supérieurs, et par ailleurs, en milieu très acide, les résultats sont moins précis car alors le peroxyde d'hydrogène commence à réagir de façon relativement significative.

On a constaté que les meilleurs résultats sont obtenus pour des valeurs de pH entre 5 et 6,3 ; cette gamme est donc préférée.

Si nécessaire, le réglage du pH est effectué par apport d'un acide tel que par exemple l'acide phosphorique ou au moyen d'un tampon, notamment un tampon phosphate.

L'iodure utilisé peut être choisi notamment parmi les iodures de métaux alcalins et l'iodure d'ammonium, toutefois l'iodure de potassium est préféré.

L'intervalle de temps entre l'ajout de l'excès d'iodure et la lecture de la couleur d'iode développée doit être toujours le même dans une série de mesures donnée, y compris au moment de l'étalonnage.

Cet intervalle de temps peut varier dans une large gamme. Il peut notamment être de l'ordre de 2 minutes à 2 minutes et demie. Il est toutefois préférable qu'il soit inférieur à 1 minute.

Par ailleurs, pour obtenir des mesures précises, il est souhaitable que la couleur d'iode formé ne soit pas trop intense. Pour ce faire, il convient que la concentration en poids du peracide dans le milieu au moment de la détermination soit de préférence inférieure à 50 ppm.

Si nécessaire, on dilue donc l'échantillon au moment de la détermination. Le liquide de dilution utilisé (eau du robinet ou eau distillée notamment) peut également contenir, si nécessaire, l'acide ou le tampon, destiné à régler le pH.

La gamme de longueurs d'ondes utilisée pour la mesure, comprise entre 430 et 500 nm, dépend essentiellement de la concentration en peracide dans le milieu au moment de la détermination.

Le système d'étalonnage à deux points intègre la droite représentant les variations de la densité optique lue en fonction de la concentration en acide peracétique.

Les deux points d'étalonnage peuvent être obtenus au moyen d'une solution de concentration connue en peracide, sans ajout d'iodure pour la valeur 0 et avec ajout d'iodure pour la valeur lue correspondant à ladite concentration connue.

Le réactif de dilution est avantageusement constitué d'eau du robinet ou éventuellement d'eau distillée, additionnée si nécessaire d'un régulateur du pH tel que par exemple l'acide phosphorique ou un tampon phosphate.

La solution d'iodure est relativement concentrée pour ne pas modifier de façon importante la dilution du flux entrant dans la cuve de mesure, par rapport à celle du flux entrant dans la cuve de référence. On peut avantageusement utiliser une solution d'iodure de potassium à une concentration de 1 à 3 % en poids.

Avantageusement, l'unité de mesure transforme la différence d'intensité en une différence de potentiel, par exemple dans la gamme de 0 à 2V, ou en intensité de courant, par exemple dans la gamme de 4-20mA.

Avantageusement, le régulateur comporte un moyen d'affichage, par exemple à cristaux liquides, de la concentration instantanée en peracide.

Avantageusement encore, le régulateur est relié à une imprimante qui reproduit les variations de la concentration.

La solution de peracide ajoutée peut être constituée d'une solution aqueuse contenant par exemple de 0,05 à 5 % en poids de peracide, notamment d'une solution aqueuse à 2,5 % en poids d'acide peracétique, lorsque celui-ci constitue le peracide dont la concentration doit être régulée.

Selon un mode préféré de réalisation, la solution de dilution et/ou de réglage de pH injectée dans le flux de mesure est mélangée intimement avec la solution à réguler, grâce à un moyen de mélange situé entre son point de rencontre avec le liquide à analyser et avant les deuxième et troisième moyens d'aspiration et qui peut être avantageusement constitué d'un étranglement suivi d'un élargissement de la tubulure dans laquelle circule le mélange.

L'étalonnage du régulateur est effectué à l'aide d'une solution étalon ou standard de concentration en peracide connue. Avantageusement, l'installation utilisée comporte un robinet à trois voies, situé avant le premier moyen d'injection et qui pemet d'introduire dans le circuit de mesure soit la solution étalon, soit la solution à réguler.

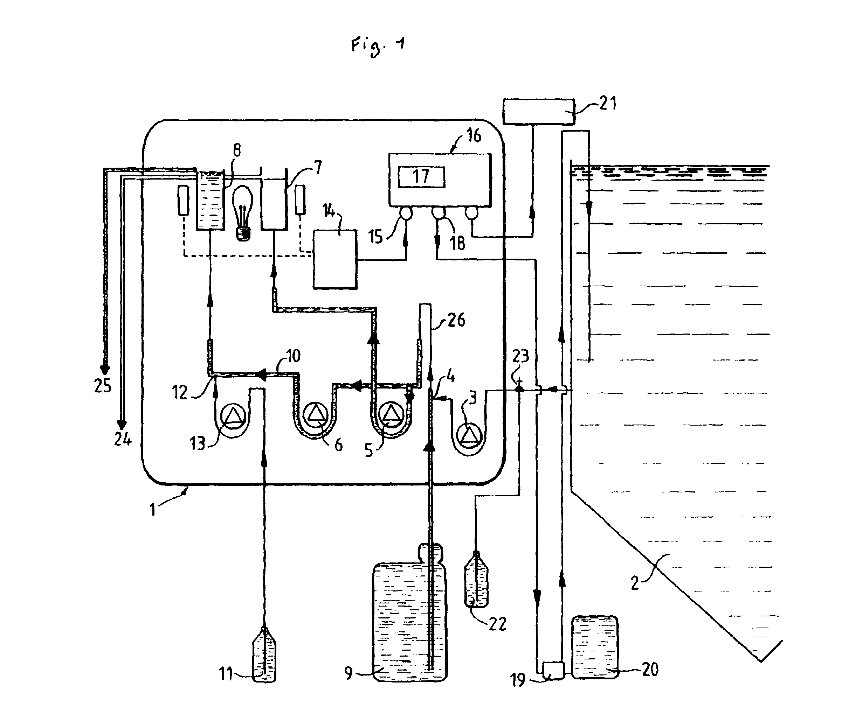

### Description de la figure unique

La figure unique annexée représente de façon schématique un mode de mise en oeuvre du procédé de régulation selon l'invention utilisant un dispositif 1 de régulation de la teneur en acide peracétique en présence de peroxyde d'hydrogène dans une solution 2 de nettoyage et/ou de désinfection, notamment pour le nettoyage en place (N.E.P.) en laiterie.

Le dispositif 1 peut être constitué par l'appareil fabriqué par la société TECAN (Suisse) et commercialisé en France par la société IMATEK sous la dénomination "Compact 150" pour réguler la concentration en chlore dans les piscines publiques avec toutefois certaines modifications, à savoir notamment le remplacement du filtre colorimétrique d'origine par un filtre ayant une bande passante de 430-470nm, ou de 450-500nm, d'une part et l'adjonction d'un système de dilution et/ou de réglage du pH (liquide 9 de dilution et/ou de réglage du pH, pompe péristaltique 3), d'autre part.

Une pompe péristaltique 3 aspire la solution à analyser et l'injecte en 4 dans le circuit d'aspiration de deux pompes péristaltiques 5 et 6 qui alimentent au même débit deux cuves d'un photomètre bi-faisceaux, à savoir la cuve de référence 7 et la cuve de mesure 8, respectivement. Ces deux pompes péristaltiques 5 et 6 ont des caractéristiques identiques et aspirent si nécessaire, outre le liquide à analyser 2 fourni par la pompe péristaltique 3, du liquide 9 de dilution et/ou de réglage du pH constitué d'eau de ville ou d'eau distillée, éventuellement additionnée d'un tampon pour ajuster le pH du flux de mesure 10 à une valeur inférieure à 6,5 permettant ainsi la réaction complète de l'acide peracétique contenu dans le liquide à analyser 2 avec l'iodure 11 en excès injecté au point 12 du flux de mesure, par l'intermédiaire de la pompe péristaltique 13. Les débits des pompes péristaltiques 3, 5 et 6 sont réglés de telle sorte que la concentration en acide peracétique du flux de mesure en 10 soit inférieure à 100 ppm. Il est avantageux d'introduire un mélangeur 26, entre le point d'injection 4 de la solution à analyser et la pompe péristaltique 5 pour homogénéiser le mélange du liquide à analyser 2 et du liquide de dilution 9. Ce mélangeur peut être notamment constitué d'un étranglement suivi d'un élargissement dans la tubulure d'alimentation comme représenté, ce qui rend le flux circulant suffisamment turbulent pour l'homogénéiser. Comme déjà indiqué plus haut, une quatrième pompe péristaltique 13 injecte dans le flux de mesure en 12 une quantité connue d'iodure 11, notamment de potasium, en excès.

Après un certain délai qui est directement fonction de la longueur du trajet entre le point d'injection 12 de l'iodure 11 et la cuve d'analyse 8, d'une part et du temps d'homogénéisation dans la cuve d'analyse 8, d'autre part, l'intensité de la couleur d'iode formée (jaune à rouge) dans la cuve d'analyse 8 est mesurée et comparée à la référence dans la cuve 7. La différence est transformée en courant (0-2V) dans l'unité de mesure 14 puis ce courant est transmis en 15 à un régulateur 16 où il est transformé en information sur un affichage à cristaux liquides 17. Ce régulateur transmet en 18 information de pilotage à la pompe 19 qui injecte si nécessaire un complément d'acide peracétique 20 dans le produit de nettoyage et/ou de désinfection 2.

Les informations fournies au régulateur 16 peuvent être transmises à une imprimante 21.

On peut étalonner le régulateur 16 sur deux points de mesure, à savoir le zéro et la concentration d'un standard ou étalon 22. En effet, la concentration en acide peracétique dans le flux de mesure 10 est ajustée de telle sorte que l'intensité de la couleur d'iode formée soit proportionnelle à la concentration en acide peracétique. Le zéro est obtenu en supprimant l'ajout d'iodure 11. Le standard ou étalon 22 est obtenu par dilution manuelle de la solution d'acide peracétique 20. L'introduction de ce standard ou étalon 22 se fait au moyen du robinet à trois voies 23. La pompe 3 aspire soit l'étalon 22 lorsqu'on effectue l'étalonnage, soit la solution à analyser 2 lorsqu'on en détermine et/ou régule la concentration en acide peracétique.

Il convient à ce propos de noter que dans certains cas particuliers (dilution et réglage du pH non nécessaires), la pompe 3 et le réactif 9 de dilution et/ou de réglage du pH peuvent être omis. Dans ce cas, les pompes 5 et 6 aspirent directement soit la solution à analyser 2, soit l'étalon 22.

Grâce à cet étalonnage, la valeur lue sur l'affichage à cristaux liquides 17 lors de la mesure est la concentration en acide peracétique dans la solution 2.

On peut ainsi programmer la valeur voulue de la concentration et la plage proportionnelle de régulation. On travaille alors avec une pompe 19 d'ajout de solution d'acide peracétique 20 qui est pilotée soit en "tout ou rien" (à n'importe quelle valeur au-dessous du point de régulation la pompe 19 se met en marche), soit en relais-contact, soit à partir d'informations dites 4-20mA, par exemple.

Ces deux dernières solutions sont plus satisfaisantes car l'inertie du système de mesure est de 5 à 10 minutes et alors la plage proportionnelle de régulation module l'information envoyée à la pompe 19.

Par ailleurs, pour plus de sécurité, le régulateur 16 peut avoir deux seuils d'alarme "haut-bas" qui sont à définir par l'utilisateur qui est ainsi prévenu si l'un de ces seuils est dépassé pendant plus d'un certain temps, lui aussi à définir.

Les liquides dcs cuves de référence 7 et d'analyse 8 sont rejetés en 24 et 25, respectivement.

### Exemples d'applications

Le dispositif décrit ci-dessus a été utilisé pour surveiller la concentration en acide peracétique d'une solution diluée de nettoyage en place (N.E.P.) en laiterie en présence de peroxyde d'hydrogène. Le but était de maintenir cette concentation à un niveau convenable pendant une semaine.

Une telle solution est envoyée dans des conduites de l'installation de la laiterie pour les désinfecter après un nettoyage acide et/ou basique. Une partie de l'acide peracétique est consommée ou hydrolysée lors de cette opération de désinfection. Il convient donc de procéder à des réajustements de la concentration en cet acide au cours du temps. Le procédé de régulation selon l'invention permet de maintenir cette solution toujours prête à l'emploi, sans intervention manuelle.

Un cycle d'une semaine a été réalisé au laboratoire dans une cuve de 30 litres de solution de désinfection, avec réglage au titre initial désiré et suivi pendant toute la semaine. Etant donné que pour différentes raisons on observe au cours du temps une diminution du volume total de la solution, un ajout de 10% d'eau a été effectué 3 fois au cours de la semaine pour observer une perte de titre plus importante que celle uniquement due à l'hydrolyse.

Le produit testé était essentiellement constitué de 25 000 ppm (en poids) d'acide peracétique, 190 000 ppm (en poids) de peroxyde d'hydrogène, en présence d'acide acétique et d'acide nitrique. Il a été utilisé ici dans des conditions habituelles d'utilisation en laiterie, à savoir en solution à 0,4%, ce qui correspondait à une concentration de 100 ppm (en poids) d'acide peracétique et de760 ppm (en poids) de peroxyde d'hydrogène dans le mélange initial.

Pour les mesures, les débits des pompes 3, 5 et 6 ont été réglés de telle sorte que l'échantillon à analyser 2 soit dilué au 1/7ème avec une eau de dilution 9 dont le pH était compris entre 5 et 6,5. Ce pH était ajusté au moyen d'acide phosphorique.

Dans une première expérience, le filtre utilisé avait une bande passante de 430-470nm.

L'étalonnage a été réalisé avec la même solution à raison de 0,5% en poids et n'a pas été modifié pendant la semaine d'essais. L'affichage à cristaux liquides au moment de l'étalonnage inscrivait 0,50.

Les résultats obtenus avec la solution étudiée sont regroupés dans le tableau I qui suit.

Dans ce tableau :
- "affichage lu" représente les valeurs lues sur l'appareil ;
- "affichage attendu" représente les valeurs obtenues par dosage manuel de l'acide peracétique (APA) dans la solution de N.E.P.

**Tableau 1**

| Jours | Affichage lu | Affichage attendu |
|---|---|---|
| 0 | 0,46 | 0,46 |
| 2 | 0,42 | 0,42 |
| 4 | 0,40 | 0,41 |
| 7 | 0,43 | 0,39 |

Conclusion : l'erreur relative entre l'affichage lu et l'affichage attendu est de 10% au maximum, ce qui est également la limite de précision du dosage manuel et est tout à fait acceptable pour une désinfection industrielle.

Une deuxième expérience a été réalisée en utilisant le même appareil, mais avec un filtre ayant une bande passante de 460 à 500nm et en utilisant la même solution que précédemment mais à 0,7% au lieu de 0,4%. L'étalonnage a été réalisé de nouveau avec cette même solution à une concentration de 0,5%.

Les résultats obtenus dans cette deuxième expérience sont regroupés dans le tableau II qui suit.

**Tableau II**

| Jours | Affichage lu | Affichage attendu |
|---|---|---|
| 0 | 0,71 | 0,71 |
| 1 | 0,70 | 0,69 |
| 2 | 0,70 | 0,68 |
| 3 | 0,72 | 0,71 |
| 4 | 0,70 | 0,67 |
| 7 | 0,72 | 0,70 |

Des essais d'interférence avec le lait ont montré que jusqu'à une concentration de 0,2% de lait entier dans la solution à analyser, aucune perturbation n'était observée sur l'appareil, alors que pour cette concentration la turbidité était de 200 unités de turbidité formazine (FTU), ce qui correspond à une turbidité relativement importante.

## Revendications

1. Procédé colorimétrique de régulation de la teneur en acide peracétique en présence de peroxyde d'hydrogène dans une solution au moins en partie aqueuse, dans laquelle le rapport molaire de la concentration en peroxyde d'hydrogène à la concentration en peracide ne dépasse pas environ 100, **caractérisé en ce que** :
- le peracide est l'acide peracétique,
- la solution à réguler (2) est aspirée par un premier moyen d'aspiration, notamment une pompe péristaltique (3), dans les circuits d'aspiration d'un deuxième et d'un troisième moyens d'aspiration, notamment des pompes péristaltiques (5, 6), fonctionnant au même débit et pouvant aspirer simultanément un liquide (9) de dilution et/ou de réglage du pH, le deuxième moyen d'aspiration (5) alimentant la cuve de référence (7) d'un photomètre bi-faisceaux et le troisième moyen d'aspiration (6) alimentant la cuve de mesure (8) dudit photomètre,
- les débits des trois moyens d'aspiration (3, 5, 6) sont réglés de telle sorte que la concentration en peracide dans le flux de mesure (10) aspiré par lesdits deuxième et troisième moyens d'aspiration (5, 6) soit inférieure à environ 100 ppm (parties par million) en poids, et que le pH du flux de mesure soit inférieur à 6,5,
- le flux de mesure (10) sortant du troisième moyen d'aspiration (6) est additionné d'un excès d'iodure (11) à l'aide d'un quatrième moyen d'aspiration, notamment une pompe péristaltique (13),
- les cellules du photomètre sont reliées à une unité de mesure (14) qui compare l'intensité de la couleur d'iode formé dans la cuve de mesure (8) à la référence dans la cuve de référence (7) et transfère la différence à un régulateur (16), lequel régulateur actionne, si nécessaire, une pompe d'injection (19) d'une solution de peracide (20) dans le milieu à réguler (2),
- la mesure est effectuée dans une gamme de longueur d'onde comprise entre 430nm et 500nm.

2. Procédé selon la revendication 1, **caractérisé en ce que** le pH au moment de la détermination et/ou régulation est réglé à une valeur comprise entre 5 et 6,3.

3. Procédé selon l'une des revendications 1 ou 2, **caractérisé en ce que** le pH est régulé si nécessaire au moyen d'acide phosphorique ou de tampon phosphate.

4. Procédé selon l'une des revendications 1 à 3, **caractérisé en ce que** l'iodure utilisé est l'iodure de potassium.

5. Procédé selon l'une des revendications 1 à 4, **caractérisé en ce que** l'intervalle de temps entre l'ajout de l'excès d'iodure et la lecture de la couleur d'iode développée est de l'ordre de 2 minutes à 2 minutes et demie, de préférence inférieur à 1 minute.

6. Procédé selon l'une des revendications 1 à 5, **caractérisé en ce que** la concentration en peracide au moment de la détermination est inférieure à 50 ppm (parties par million) en poids.

7. Procédé selon l'une des revendications 1 à 6, **caractérisé en ce que** l'unité de mesure (14) transforme la différence d'intensité en une différence de potentiel, notamment dans la gamme de 0 à 2V, ou en intensité de courant, notamment dans la gamme de 4-20mA.

8. Procédé selon l'une des revendications 1 à 7, **caractérisé en ce que** le régulateur (16) comporte un moyen d'affichage (17), notamment à cristaux liquides, de la concentration instantanée en peracide.

9. Procédé selon l'une des revendications 1 à 8, **caractérisé en ce que** la solution de peracide (20) est consituée d'une solution aqueuse contenant de 0,05 à 5 % en poids de peracide, notamment d'une solution aqueuse de 2,5 % en poids d'acide peracétique, lorsque celui-ci constitue le peracide dont la concentration doit être régulée.

10. Procédé selon l'une des revendications 1 à 9, **caractérisé en ce que** la solution (9) de dilution et/ou de réglage du pH injectée dans le flux de mesure (10) est mélangée intimement avec la solution à réguler (2), grâce à un moyen de mélange (26) situé entre son point de rencontre (4) avec le liquide à analyser (2) et avant les deuxième et troisième moyens d'aspiration (5, 6).

## Patentansprüche

1. Kolorimetrisches Verfahren zur Einstellung des Persäuregehalts in Gegenwart von Wasserstoffperoxid in einer Lösung mit mindestens einem wäßrigen Anteil, in der das molare Verhältnis der Wasserstoffperoxidkonzentration zur Persäurekonzentration nicht mehr als etwa 100 beträgt, **dadurch gekennzeichnet, daß**
- es sich bei der Persäure um Peressigsäure handelt,
- die einzustellende Lösung (2) mit Hilfe eines ersten Ansaugmittels, insbesondere einer peristaltischen Pumpe (3), in die Ansaugkreisläufe eines zweiten und eines dritten Ansaugmittels, insbesondere peristaltischer Pumpen (5, 6), die mit der gleichen Durchflußrate arbeiten und gleichreitig eine Flüssigkeit (9) zur Verdünnung und/oder zur Einstellung des pH-Werts ansaugen können, angesaugt wird, wobei das zweite Ansaugmittel (5) die Referenzküvette (7) eines Zweistrahlphotometers speist und das dritte Ansaugmittel (6) die Meßküvette (8) des Photometers speist,
- die Durchflußraten der drei Ansaugmittel (3, 5, 6) so eingestellt werden, daß die Persäurekonzentration in dem mit Hilfe des zweiten und des dritten Ansaugmittels (5, 6) angesaugten Meßfluß (10) kleiner als etwa 100 Gew.-ppm (Gewichtsteile pro Million) ist und der pH-Wert des Meßflusses kleiner als 6,5 ist,
- der aus dem dritten Ansaugmittel (6) austretende Meßfluß (10) mit Hilfe eines vierten Ansaugmittels, insbesondere einer peristaltischen Pumpe (13), mit einem Iodidüberschuß (11) versetzt wird,
- die Photometerzellen mit einer Meßeinheit (14) verbunden werden, die die Farbintensität von in der Meßküvette (8) gebildetem Iod mit der Referenz in der Referenzküvette (7) vergleicht und die Differenz an einen Regler (16) weitergibt, wobei der Regler gegebenenfalls eine Pumpe (19) zum Einleiten einer Persäurelösung (20) in das einzustellende Medium (2) in Betrieb setzt, und
- die Messung in einem Wellenlängenbereich zwischen 430 nm und 500 nm erfolgt.

2. Verfahren nach Anspruch 1, **dadurch gekennzeichnet, daß** der pH-Wert zum Zeitpunkt der Bestimmung und/oder Einstellung auf einen Wert zwischen 5 und 6.3 eingestellt wird.

3. Verfahren nach Anspruch 1 oder 2, **dadurch gekennzeichnet, daß** der pH-Wert gegebenenfalls mit Phosphorsäure oder Phosphatpuffer eingestellt wird.

4. Verfahren nach einem der Ansprüche 1 bis 3, **dadurch gekennzeichnet, daß** man als Iodid Kaliumiodid verwendet.

5. Verfahren nach einem der Ansprüche 1 bis 4, **dadurch gekennzeichnet, daß** sich die Zeitspanne zwischen der Zugabe des Iodidüberschusses und dem Ablesen der entwickelten Iodfarbe auf etwa 2 bis 2,5 Minuten, vorzugsweise weniger als 1 Minute, beläuft.

6. Verfahren nach einem der Ansprüche 1 bis 5, **dadurch gekennzeichnet, daß** die Persäurekonzentration zum Zeitpunkt der Bestimmung kleiner als 50 Gew.-ppm (Gewichtsteile pro Million) ist.

7. Verfahren nach einem der Ansprüche 1 bis 6, **dadurch gekennzeichnet, daß** die Meßeinheit (14) die Intensitätsdifferenz in eine Potentialdifferenz, insbesondere im Bereich von 0 bis 2 V, oder in eine Stromstärke, insbesondere im Bereich von 4 bis 20 mA, umwandelt.

8. Verfahren nach einem der Ansprüche 1 bis 7, **dadurch gekennzeichnet, daß** der Regler (16) ein Anzeigemittel (17), insbesondere eine Flüssigkristallanzeige, für die momentane Persäurekonzentration umfaßt.

9. Verfahren nach einem der Ansprüche 1 bis 8, **dadurch gekennzeichnet, daß** die Persäurelösung (20) aus einer wäßrigen Lösung mit 0,05 bis 5 Gew.-% Persäure insbesondere aus einer wäßrigen Lösung mit 2,5 Gew.-% Peressigsäure besteht, wenn diese die Persäure darstellt, deren Konzentration eingestellt werden soll.

10. Verfahren nach einem der Ansprüche 1 bis 9, **dadurch gekennzeichnet, daß** die in den Meßfluß (10) eingeleitete Lösung (9) zur Verdünnung und/oder zur Einstellung des pH-Werts mit einer Mischeinrichtung (26), die zwischen ihrem Treffpunkt (4) mit der zu analysierenden Flüssigkeit (2) und vor dem zweiten und dem dritten Ansaugmittel (5, 6) angeordnet ist, innig mit der einzustellenden Lösung (2) vermischt wird.

## Claims

1. Colorimetric method for regulating the peracetic acid content, in the presence of hydrogen peroxide, in an at least partly aqueous solution in which the molar ratio of the hydrogen peroxide concentration to the peracid concentration does not exceed approximately 100, **characterized in that**:
- the peracid is peracetic acid,
- the solution to be adjusted (2) is aspirated by a first means of aspiration, more particularly a peristaltic pump (3), into the aspiration circuits of a second and third means of aspiration, more particularly peristaltic pumps (5, 6), operating at the same flow rate and being able to aspirate simultaneously a dilution liquid (9) and/or a liquid for adjusting the pH, the second means of aspiration (5) supplying the reference cell (7) of a two-beam photometer and the third means of aspiration (6) supplying the measuring cell (8) of the said photometer,
- the flow rates of the three means of aspiration (3, 5, 6) are adjusted so that the peracid concentration in the flow measured (10) aspirated by the said second and third means of aspiration (5, 6) is less than approximately 100 ppm (parts per million) by weight, and that the pH of the flow measured is less than 6.5,
- an excess of iodide (11) is added to the flow measured (10) leaving the third means of aspiration (6) with the aid of a fourth means of aspiration, more particularly a peristaltic pump (13),
- the photometer cells are connected to a measuring unit (14) which compares the intensity of the iodine colour formed in the measuring cell (8) with the reference colour in the reference cell (7) and transfers the difference to a regulator (16), which regulator actuates, if necessary, a pump (19) injecting a solution of peracid (20) into the medium to be adjusted (2),
- the measurement is carried out in a wavelength range of between 430 nm and 500 nm.

2. Method according to Claim 1, **characterized in that** the pH at the moment of the determination and/or adjustment is adjusted to a value of between 5 and 6.3.

3. Method according to either of Claims 1 and 2, **characterized in that** the pH is adjusted if necessary by means of phosphoric acid or a phosphate buffer.

4. Method according to one of Claims 1 to 3, **characterized in that** the iodide used is potassium iodide.

5. Method according to one of Claims 1 to 4, **characterized in that** the interval of time between adding the excess of iodide and reading the iodine colour developed is of the order of two minutes to two and a half minutes, preferably less than one minute.

6. Method according to one of Claims 1 to 5, **characterized in that** the peracid concentration at the moment of the determination is less than 50 ppm (parts per million) by weight.

7. Method according to one of Claims 1 to 6, **characterized in that** the measuring unit (14) converts the difference in intensity into a difference in potential, more particularly in the range of 0 to 2 V, or to a current intensity, more particularly within the range of 4-20 mA.

8. Method according to one of Claims 1 to 7, **characterized in that** the regulator (16) comprises a means of display (17), more particularly a liquid crystal display, showing the instantaneous concentration of peracid.

9. Method according to one of Claims 1 to 8, **characterized in that** the peracid solution (20) consists of an aqueous solution containing 0.05 to 5% by weight of peracid, more particularly an aqueous solution containing 2.5% by weight of peracetic acid, when the latter constitutes the peracid of which the concentration is to be adjusted.

10. Method according to one of Claims 1 to 9, **characterized in that** the solution (9) for diluting and/or adjusting the pH injected into the flow measured (10) is mixed intimately with the solution to be adjusted (2), using a mixing means (26) situated between the point (4) at which it encounters the liquid to be analysed (2) and before the second and third means of aspiration (5, 6).
